# EUROPÄISCHE PATENTANMELDUNG

(11) **EP 4 378 386 A1**
(43) Veröffentlichungstag der Anmeldung: **05.06.2024**
(21) Anmeldenummer: 23161318.3
(22) Anmeldetag: 10.03.2023
(51) Int. Cl.: A61B 5/145, A61B 5/1455, A61B 5/00

(54) **SENSORIK UND WC-SITZGARNITUR**

(30) Priorität: 01.12.2022 DE 102022131893; 29.12.2022 DE 102022134969
(71) Anmelder: Hamberger Industriewerke GmbH, 83071 Stephanskirchen (DE)
(72) Erfinder:
(74) Vertreter: Winter, Brandl - Partnerschaft mbB

(57) **Zusammenfassung**

Offenbart sind eine Sensorik (12) zur nicht-invasiven Erfassung des Glucosegehalts im Blutkreislauf einer Person und eine mit einer derartigen Sensorik (12) ausgeführte WC-Sitzgarnitur (1).

## Beschreibung

Die Erfindung betrifft eine Sensorik zur nicht-invasiven Erfassung des Glucosegehalts gemäß dem Oberbegriff des Patentanspruches 1 und eine mit einer derartigen Sensorik ausgeführte WC-Sitzgarnitur.

Diabetes ist eine weltweit stark verbreitete Autoimmunerkrankung, bei der die Insulinproduktion in der Bauchspeicheldrüse beeinträchtigt ist, so dass der Blutzuckerspiegel erhöht ist. Bei Patienten mit Typ-I-Diabetes oder Typ-II-Diabetes wird der Blutzuckerspiegel kontrolliert, so dass dieser ggf. bei einer Überhöhung durch Insulininjektion abgesenkt werden kann. Die Überwachung des Blutzuckerspiegels erfolgt dabei üblicherweise durch eine Analyse des Bluts, wobei eine Nadel in den Finger gestochen wird und dann ein aus dieser Verletzung resultierender Blutstropfen auf einen Teststreifen aufgebracht und dieser in ein Blutzuckermessgerät eingelegt wird, das dann den Blutzuckergehalt anzeigt. Dieses Messverfahren ist relativ aufwendig und zudem bei nicht sachgemäßer Durchführung schmerzhaft, wobei auch die Gefahr besteht, dass sich der Einstechbereich der Haut durch Verschmutzungen entzündet.

Als Alternative zu diesem sehr aufwendigen und für den Patienten unangenehmen Messverfahren wird in dem europäischen Patent EP 3 155 401 B1 ein Messgerät vorgeschlagen, das eine nicht-invasive Blutzuckermessung ermöglicht.

Ähnlich wie bei den herkömmlichen invasiven Messverfahren ist es auch bei dieser Vorgehensweise erforderlich, dass die Patienten jeweils ein geeignetes Messgerät mit sich führen, so dass ein erheblicher Aufwand zur Durchführung der Messung erforderlich ist.

Demgegenüber liegt der Erfindung die Aufgabe zugrunde, die nicht-invasive Erfassung des Glucosegehalts im Blut zu vereinfachen.

Diese Aufgabe wird durch eine Sensorik mit den Merkmalen des Patentanspruches 1 gelöst. Zur erfindungsgemäßen Lösung gehört auch eine WC-Sitzgarnitur mit einer derartigen Sensorik.

Vorteilhafte Weiterbildungen der Erfindung sind Gegenstand der Unteransprüche.

Die erfindungsgemäße Sensorik dient zur nicht-invasiven Erfassung des Glucosegehalts im Blutkreislauf eines Patienten, wobei die Sensorik erfindungsgemäß so ausgelegt ist, dass sie in eine WC-Sitzgarnitur integrierbar ist. Diese ist - in an sich bekannter Weise - mit einem WC-Sitz und einem WC-Deckel ausgeführt, die mittels einer WC-Sitzgelenkanordnung gelenkig verbunden sind. Die Sensorik ist so ausgelegt, dass sie in den WC-Sitz und/oder den WC-Deckel aufgenommen werden kann. Auch eine abschnittsweise Aufnahme in einer Technikbox ist möglich, in der auch beispielsweise eine Energieversorgung vorgesehen sein kann.

Eine derartige Sensorik ermöglicht es einem oder aufeinanderfolgend mehreren Patienten, die Glucosegehaltmessung mit minimalem Aufwand auf dem WC während des bestimmungsgemäßen Gebrauchs durchzuführen.

WC-Sitzgarnituren mit Sensoren, die die Erfassung von Vitalparametern ermöglichen, sind beispielsweise aus der Patentanmeldung EP 3 960 054 A1 oder WO 2014/095 958 A1 der Anmelderin bekannt. Dieser Stand der Technik betrifft allerdings lediglich Sensoren, die zur Erfassung von herkömmlichen Vitalparametern, wie dem Körpergewicht oder einem EKG geeignet sind. Eine Erfassung des Glucosegehalts ist nicht näherungsweise angesprochen. Selbstverständlich können derartige Sensoren auch in die WC-Sitzgarnitur integriert sein, so dass mehrere Vitalparameter während einer WC-Nutzung erfassbar sind.

Gemäß einem bevorzugten Ausführungsbeispiel der Erfindung hat die Sensorik einen Sensor, der in den Sitz oder den Deckel integriert ist und dem eine vorzugsweise ebenfalls im Sitz oder Deckel aufgenommene Auswerteeinheit zugeordnet ist. Diese Ausführungsform ermöglicht es, den Glucosewert über den Sensor zu erfassen und dann auch über die integrierte Auswerteeinheit auszuwerten.

Dabei ist es besonders bevorzugt, wenn die Auswerteeinheit ein Funkmodul oder eine sonstige Einrichtung zur, insbesondere drahtlosen, Übertragung von Messwerten hat. Dabei kann die Übertragung beispielsweise an ein Display oder einen Zentralrechner erfolgen.

Bei einer Variante der Erfindung ist der Sensor ausgelegt, die Glucosemoleküle im Blutkreislauf einer die WC-Sitzgarnitur nutzenden Person zu erfassen.

Ein derartiger Sensor kann als optischer Sensor ausgeführt sein, wobei das ausgestrahlte Licht ggf. die Wandung des Sitzes/Deckels durchdringt und auch den Bereich der Haut, der auf diesen Komponenten positioniert ist. Selbstverständlich sind auch auf anderen Wirkprinzipien basierende Sensoren einsetzbar, die vorzugsweise so ausgeführt sind, dass sie ein Messignal durch eine Deckschicht des Sitzes oder Deckels hindurch aussenden bzw. empfangen können.

Bei einem besonders bevorzugten Ausführungsbeispiel ist der WC-Sitz oder der WC-Deckel mit einem Hohlraum ausgeführt, der durch zumindest zwei Schalen begrenzt wird und in den die Sensorik integriert ist.

Dabei kann die Sensorik so ausgeführt sein, dass sie durch eine Schalenwandung/Deckschicht hindurch Messsignale aussendet oder empfängt. Dabei ist es besonders bevorzugt, wenn die Schalenwandung/Deckschicht einen Fensterbereich mit optimierter Durchlässigkeit für die Sensor-Messsignale hat.

Die Sensorik kann so ausgelegt sein, dass bei einer unzureichenden Positionierung einer das WC nutzenden Person oder einer fehlerbehafteten Messwerterfassung ein Warnsignal abgegeben wird.

Bei einem bevorzugten Ausführungsbeispiel der Erfindung ist zumindest der Sensor im Schenkelauflagebereich des WC-Sitzes positioniert.

Bevorzugte Ausführungsbeispiele der Erfindung werden im Folgenden anhand schematischer Zeichnungen näher erläutert. Es zeigen:
- Figur 1: eine Seitenansicht einer erfindungsgemäßen WC-Sitzgarnitur und
- Figur 2: eine Einzeldarstellung eines WC-Sitzes der WC-Sitzgarnitur gemäß Figur 1 mit einer erfindungsgemäßen Sensorik.

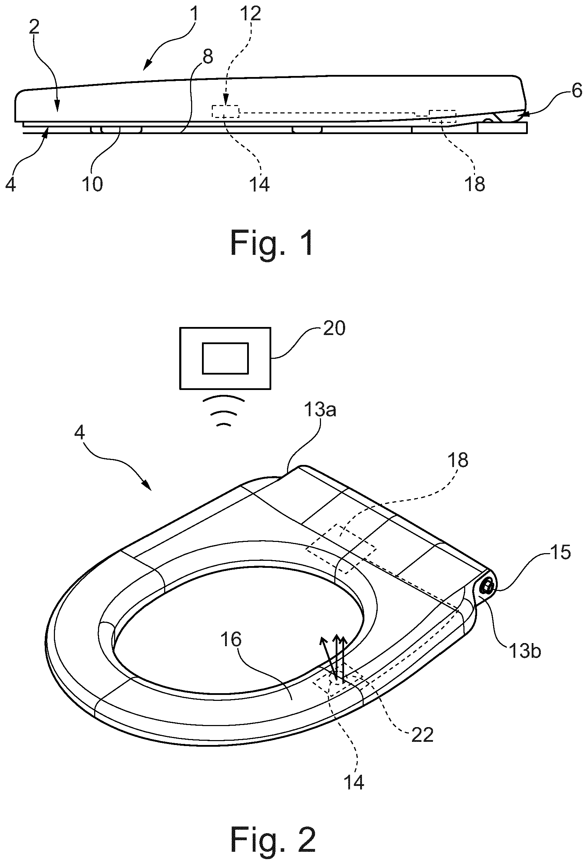

Figur 1 zeigt eine Seitenansicht eines Ausführungsbeispiels einer erfindungsgemäßen WC-Sitzgarnitur 1, die in an sich bekannter Weise mit einem WC-Deckel 2 und einem in der Darstellung gemäß Figur 1 von diesem weitgehend überdeckten WC-Sitz 4 ausgeführt ist. Die beiden Komponenten sind über eine WC-Sitzgelenkanordnung 6 schwenkbar mit einander verbunden, wobei diese über geeignete Befestigungsmittel auf einer Keramik 8 befestigt ist. In der dargestellten Schließposition liegt dabei der Sitz 4 über Puffer 10 auf der Keramik 8 auf. Wie in Figur 1 angedeutet, ist die WC-Sitzgarnitur 1 mit einer Sensorik 12 ausgeführt, die vorzugsweise in den WC-Sitz 4 integriert ist.

Dies erschließt sich insbesondere aus Figur 2, die eine Einzeldarstellung des WC-Sitzes 4 der WC-Sitzgarnitur 1 zeigt. Bei diesem Ausführungsbeispiel hat der WC-Sitz 4 rückseitig (rechts in Figur 2) einen durchgehenden oder zwei zu einander beabstandete Kloben 13a, 13b, aus denen nach außen (in Figur 2 ist nur eine Seite sichtbar) Rotationskolben 15 eines Dämpfers auskragen, der jeweils über die Sitzgelenkanordnung 6 auf der Keramik 8 abgestützt ist. Beim dargestellten Ausführungsbeispiel sind die Kloben 13a, 13b aus Designgründen mittels eines Wandungsabschnitts mit einander verbunden. Die Endabschnitte der Rotationskolben 15 greifen in an sich bekannter Weise auch in einen Kloben des in Figur 2 nicht dargestellten WC-Deckels 2 derart ein, so dass sowohl die Absenkbewegung des WC-Deckels 2 als auch die des WC-Sitzes 4 über die beiden Dämpfer gedämpft wird.

Beim Ausführungsbeispiel gemäß Figur 2 ist die Sensorik 12 im wesentlichen vollständig in dem WC-Sitz 4 aufgenommen. Dieser hat einen zweischaligen Aufbau, wie er beispielsweise in der vorgenannten Druckschrift EP 3 960 054 A1 offenbart ist, so dass weitere Erläuterungen eines derartigen Schalenaufbaus durch Verweis auf diese Druckschrift entbehrlich sind. Durch diesen zweischaligen Aufbau wird ein Hohlraum ausgebildet, in den die Sensorik 12 eingesetzt ist. Wie in Figur 2 angedeutet, hat die Sensorik 12 einen Sensor 14, der in einem Bereich 16 des Hohlraums positioniert ist, oberhalb dem die Schenkel eines Nutzers/Patienten auf dem WC-Sitz 4 aufliegen. Dieser Sensor 14 steht über eine Schaltung oder kabellos in Wirkverbindung mit einer elektronischen Auswerteeinheit 18, die ausgelegt ist, die vom Sensor 14 erfassten Messwerte zu verarbeiten und ggf. über ein Funkmodul an ein Display oder einen Zentralrechner, beispielsweise ein Tablet 20, zu senden, so dass der Nutzer über das Messergebnis informiert ist. Die erfindungsgemäße WC-Sitzgarnitur 1 kann auch mit einer Identifikationseinrichtung ausgeführt sein, über die der jeweilige Nutzer identifiziert werden kann, so dass die entsprechenden Messwerte personenbezogen abgelegt und ausgelesen werden können. Das Tablet 20 kann dabei so ausgeführt sein, dass personenbezogene Daten lediglich nach Eingabe einer PIN oder dergleichen möglich sind.

Bei einem Ausführungsbeispiel der Erfindung ist der Sensor 14 so ausgelegt, dass er Licht im nicht sichtbaren Bereich abstrahlt, das die Schalenwandung durchsetzt. Im Prinzip kann in dem Abstrahlbereich ein Fenster 22 vorgesehen sein, das beispielsweise durch einen mit geringerer Wandstärke ausgeführten Bereich der Schale oder durch einen aus einem anderen Material bestehenden Bereich der Schale ausgeführt ist, so dass das abgestrahlte Licht nicht oder nur wenig geschwächt wird. Über das vom Sensor 14 ausgesendete und vom Blut des Nutzers reflektierte Licht ist es beispielsweise möglich, die Glucosemoleküle im Blut zu zählen und daraus auf den Glucosegehalt zu schließen. Wie gesagt, können auch auf anderen Wirkprinzipien funktionierende Sensoren genutzt werden.

Das vorgenannte Fenster 22 kann so ausgestaltet sein, dass es von außen, d.h. aus der Sicht der die WC-Sitzgarnitur nutzenden Person nicht sichtbar ist. So kann der Sensor 14 nutzerseitig von einer Deckschicht überdeckt sein, deren Aufbau und Design so ausgelegt ist, dass sie für Messignale, bspw. Sende- und Empfangssignale der Sensorik 12 durchlässig ist, aber in der optischen Anmutung an das Design der umliegenden Flächenbereiche angepasst ist. Natürlich kann auch zur Vereinfachung der Nutzung die Position des Sensors 14 gekennzeichnet sein. Die vorbestimmte Positionierung der nutzenden Person mit Bezug zum Sensor 14 kann auch über die Auswerteeinheit 18 erfasst werden, so dass beispielsweise bei falscher Positionierung ein Warnsignal abgegeben wird.

Offenbart sind eine Sensorik zur nicht-invasiven Erfassung des Glucosegehalts im Blutkreislauf einer Person und eine mit einer derartigen Sensorik ausgeführte WC-Sitzgarnitur.

### Bezugszeichenliste:

- 1: WC-Sitzgarnitur
- 2: WC-Deckel
- 4: WC-Sitz
- 6: Sitzgelenkanordnung
- 8: Keramik
- 10: Puffer
- 12: Sensorik
- 13: Kloben
- 14: Sensor
- 15: Rotationskolben
- 16: Schenkelauflagebereich
- 18: Auswerteeinheit
- 20: Tablet
- 22: Fenster

## Patentansprüche

1. Sensorik zur nicht-invasiven Erfassung des Glucosegehalts im Blutkreislauf einer Person, **dadurch gekennzeichnet, dass** die Sensorik (12) ausgelegt ist, in eine WC-Sitzgarnitur (1) integriert zu sein, die mit einem WC-Sitz (4) und einem WC-Deckel (2) ausgeführt ist, die mittels einer WC-Sitzgelenkanordnung 6 gelenkig verbunden sind, wobei die Sensorik (12) derart ausgeführt ist, dass sie in dem WC-Sitz (4) und/oder dem WC-Deckel (2) positionierbar ist.

2. Sensorik nach Patentanspruch 1, mit einem Sensor (14), der ausgelegt ist, in den WC-Sitz (4) oder den WC-Deckel (2) integriert zu werden und dem eine vorzugsweise ebenfalls im WC-Sitz (4) oder im WC-Deckel (2) aufnehmbare Auswerteeinheit (18) zugeordnet ist.

3. Sensorik nach Patentanspruch 2, wobei die Auswerteeinheit (18) ein Funkmodul zur Übertragung von Messwerten an ein Display oder einen Rechner hat.

4. Sensorik nach Patentanspruch 2 oder 3, wobei der Sensor (14) ausgelegt ist, Glucosemoleküle im Blutkreislauf einer die WC-Sitzgarnitur (1) nutzenden Person zu erfassen.

5. Sensorik nach Patentanspruch 4, wobei der Sensor (14) ein nicht-invasiver Sensor, vorzugsweise ein optischer Sensor ist.

6. WC-Sitzgarnitur mit einem WC-Sitz (4) und einem WC-Deckel (2), die mittels einer WC-Sitzgelenkanordnung (6) gelenkig verbunden sind, **gekennzeichnet durch** eine Sensorik (12) nach einem der vorhergehenden Patentansprüche.

7. WC-Sitzgarnitur nach Patentanspruch 6, wobei der WC-Sitz (4) oder der WC-Deckel (2) einen durch zumindest zwei Schalen gebildeten Hohlraum hat, in dem die Sensorik (12) aufgenommen ist.

8. WC-Sitzgarnitur nach Patentanspruch 7, wobei der Sensor (14) durch eine Schalenwandung oder Deckschicht hindurch abstrahlt.

9. WC-Sitzgarnitur nach Patentanspruch 8, wobei die Schalenwandung oder die Deckschicht, vorzugsweise als ein Fenster (22), mit einer optimierten Durchlässigkeit für Messsignale ausgeführt ist.

10. WC-Sitzgarnitur nach einem der Patentansprüche 6 bis 9, wobei der Sensor (14) unterhalb eines Schenkelauflagebereichs (16) des WC-Sitzes (4) positioniert ist.
